(19) Europäisches Patentamt
European Patent Office
Office européen des brevets

(11) **EP 1 785 164 B1**

(12) **EUROPÄISCHE PATENTSCHRIFT**

(45) Veröffentlichungstag und Bekanntmachung des Hinweises auf die Patenterteilung:
**19.08.2009 Patentblatt 2009/34**

(51) Int Cl.:
*A61N 7/00* *(2006.01)*    *A61B 17/00* *(2006.01)*
*A61B 17/22* *(2006.01)*

(21) Anmeldenummer: **06123863.0**

(22) Anmeldetag: **10.11.2006**

(54) **Vorrichtung zur Einstrahlung von Ultraschall in Gewebe**

Apparatus for irradiating ultrasound into tissue

Dispositif pour irradier des ultrasons dans un tissu

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **11.11.2005 DE 102005053918**

(43) Veröffentlichungstag der Anmeldung:
**16.05.2007 Patentblatt 2007/20**

(73) Patentinhaber: **Zimmer MedizinSysteme GmbH 89231 Neu-Ulm (DE)**

(72) Erfinder: **Zimmer, Bernd 89231, Neu-Ulm (DE)**

(74) Vertreter: **Peckmann, Ralf Reinhard, Skuhra, Weise & Partner GbR Patent- und Rechtsanwälte Friedrichstrasse 31 80801 München (DE)**

(56) Entgegenhaltungen:
**US-A- 5 413 550       US-A- 5 601 526
US-A1- 2004 082 857**

**Beschreibung**

Die vorliegende Erfindung betrifft eine Vorrichtung zum Einstrahlen von Ultraschall in zu behandelnde Gewebeteile, wobei Ultraschallpulse verwendet werden.

Ultraschall wird in der Medizintechnik einerseits als diagnostisches Hilfsmittel bei bildgebenden Verfahren verwendet und andererseits auch therapeutisch eingesetzt. Ultraschall erzeugt im Gewebe Dichtewellen, die als Vibrationen und als Wärme wirksam werden. Durch Ultraschalleinstrahlung wird somit einerseits eine mechanische Wirkung erzielt, die wie Mikromassagen im Weichteilgewebe wirkt und dabei beispielsweise die Freisetzung von Gewebehormonen stimuliert und Einfluss auf Stoffwechsel und Muskelzustand hat. Die anregende Wirkung dieser mechanischen Komponente von therapeutischem Ultraschall kann positiven Einfluss auf eine Geweberegeneration haben. Die thermische Komponente von therapeutischem Ultraschall führt zur Gewebeerwärmung, die beispielsweise bei der Thermotherapie ausgenutzt wird.

Therapeutischer Ultraschall kommt sowohl kontinuierlich wie auch in gepulster Form zur Anwendung. Bei kontinuierlicher Einstrahlung erzeugt ein geeigneter Schwingungserzeuger kontinuierlich Ultraschallwellen mit einer vorgegebenen Ultraschallfrequenz. Bei gepulstem Ultraschall werden Pulse von Ultraschall erzeugt. Diese Pulse von Ultraschall weisen dann eine zeitliche Pulsbreite oder -Länge auf, während der Ultraschall der jeweiligen Ultraschallfrequenz eingestrahlt wird.

Darauf folgt ein Zeitintervall mit einer Einstrahlungspause, in dem keinerlei Einstrahlung erfolgt. Die Anzahl derartiger Ultraschallpulse pro Zeiteinheit ergeben eine Pulsfrequenz. Im Grenzfall verschwindender Einstrahlungspausen ergibt sich kontinuierlicher Ultraschall.

Die therapeutische Wirkung des eingestrahlten Ultraschalls hängt dabei insbesondere von der ausgewählten Ultraschallfrequenz, der Dauer der Anwendung und auch von der Art der eingestrahlten Ultraschallpulse ab. Als Pulsparameter kommen zum Beispiel die Pulsbreite, Pausenlänge, Ultraschallfrequenz, die Amplitude des Ultraschalls und die Pulsfrequenz in Frage. Der Therapeut muss dabei abwägen, welche Leistung und in welcher Signalform der therapeutische Ultraschall eingesetzt werden muss, was im Einzelfall schwer zu beurteilen ist. Auch eine präzise Einstellung der Behandlungstiefe in einem Gewebe durch Ultraschallapplikation ist häufig schwierig.

In der Vergangenheit wurde beispielsweise vorgeschlagen, gleichzeitig mittels mehrerer Ultraschallsender Ultraschallenergie mit verschiedenen Ultraschallfrequenzen in ein zu behandelndes Gewebe einzustrahlen. Die DE 103 06 795 A1 offenbart eine entsprechende Ultraschalleinrichtung, bei der mehrere Ultraschallstrahlen verschiedener Frequenzen gleichzeitig in einem gemeinsamen Fokusbereich im Gewebe wirken. Nachteilig ergibt sich jedoch ein hoher Implementierungsaufwand mit mehreren Ultraschallquellen und die Bedienung durch den Therapeuten, der selbstständig viele Parameter wie Frequenz, Pulslängen und Einstrahlleistung festlegen muss.

Das Dokument US 2004/0082857 offenbart eine Vorrichtung gemäß dem Oberbegriff der Ansprüche 1 und 9.

Es ist daher eine Aufgabe der vorliegenden Erfindung, ein Verfahren zum Einstrahlen von Ultraschall zu schaffen, welches besonders einfach durch einen Therapeuten an die jeweiligen therapeutischen Anforderungen anpassbar ist. Ferner soll eine flexible Einstellung der Behandlungstiefe möglich sein.

Diese Aufgabe wird durch eine Vorrichtung zum Einstrahlen von Ultraschall in ein Gewebe gemäß Patentanspruch 1 gelöst, und ferner löst diese Aufgabe eine Vorrichtung zur Einstrahlung von Ultraschall in Gewebe mit den Merkmalen des Patentanspruchs 9.

Demgemäß wird eine Vorrichtung zur Einstrahlung von Ultraschall in ein Gewebe mit einer vorgegebenen thermischen Wirkung und einer vorgegebenen mechanischen Wirkung in dem Gewebe vorgeschlagen, wobei nacheinander Ultraschallpulse mit einer jeweiligen Pulsbreite eingestrahlt werden, und wobei ein Tastverhältnis der Ultraschallpulse in Abhängigkeit von der thermischen und der mechanischen Wirkung des Ultraschalls eingestellt wird.

Erfindungsgemäß kann der jeweilige Therapeut gemäß seinen Therapievorstellungen sowohl eine thermische Wirkung wie auch eine gewünschte mechanische Wirkung in dem zu behandelnden Gewebe vorgeben. Erfindungsgemäß werden dann sequentiell Ultraschallpulse in das Gewebe eingestrahlt, wobei insbesondere deren Tastverhältnis, also bei einer periodisch pulsierten Abgabe von Ultraschall, das Verhältnis der Periodendauer zum Zeitraum, in dem Ultraschallleistung eingestrahlt wird, automatisch bestimmt werden. Als Einstrahlungsmittel sind Ultraschallköpfe mit geeigneten Schwingungserzeugern bekannt. Häufig werden Schallköpfe mit Piezo-Schwingungserzeugern verwendet.

In einer bevorzugten Ausführungsform der erfindungsgemäßen Vorrichtung werden die folgenden Schritte von einer Steuereinrichtung durchgeführt:

- Vorgeben eines mechanischen Wirkungsparameters für die mechanische Wirkung;

- Vorgeben eines thermischen Wirkungsparameter für die thermische Wirkung;

- Festlegen des Tastverhältnisses in Abhängigkeit von dem thermischen Wirkungsparameter und von dem mechanischen Wirkungsparameter; und

- Aktivieren und Deaktivieren des Einstrahlungsmittels zum Einstrahlen von Ultraschallpulsen mit dem festgelegten Tastverhältnis.

Durch das Einschalten und Ausschalten bzw. Aktivieren und Deaktivieren des Einstrahlungsmittels werden erfindungsgemäße Pulsbreiten und Abfolgen erreicht.

Dabei wird bevorzugt als mechanischer Wirkungsparameter eine Amplitude der abgegebenen Ultraschallleistung verwendet. Diese wird in der Regel in $W/cm^2$ angegeben und hängt im Wesentlichen von der Amplitude der Schallwellen ab. Die biologische Wirkung dieser mechanischen Komponente des Ultraschalls beruht auf reversibler Mikrokavitation und Flüssigkeitsverschiebungen in dem Gewebe. Die Amplitude ist daher ein günstiger mechanischer Wirkungsparameter, welcher für einen Therapeuten anschaulich verständlich ist.

Als thermischer Wirkungsparameter wird vorzugsweise eine effektiv in das Gewebe abgegebene Ultraschallleistung verwendet. Die zumeist in Reibung umgesetzte Leistung des eingestrahlten Ultraschalls führt zu vermehrter Braun'scher Bewegung und molekularer Reibung, wodurch im Gewebe eine Temperaturerhöhung erfolgt. Die jeweilige Erwärmung hängt neben der Amplitude der Ultraschallwellen auch von der Frequenz und der eingebrachten Gesamtenergie und auch von der Anwendungsdauer ab.

Aus der vorgegebenen mechanischen Wirkung oder dem entsprechenden Wirkungsparameter wird vorzugsweise eine maximal mögliche thermische Wirkung ermittelt.

In einer Weiterbildung der erfindungsgemäßen Vorrichtung zum Einstrahlen von Ultraschall weisen die Ultraschallpulse unterschiedliche Ultraschallfrequenzen auf. Die somit nacheinander mit einem jeweiligen erfindungsgemäß bestimmten Tastverhältnis eingestrahlten Ultraschallpulse erlauben eine besonders präzise Bestimmung der Eindringtiefe und damit eine einfach einstellbare Therapieform hinsichtlich der thermischen, mechanischen und lokalen Wirkung des Ultraschalls.

Eine alternative Ausführung der erfindungsgemäßen Vorrichtung zur Einstrahlung von Ultraschall in ein Gewebe mit einer vorgegebenen thermischen Wirkung in einer vorgegeben Gewebetiefe in dem Gewebe sieht vor, dass nacheinander Ultraschallpulse mit einer jeweiligen Pulsbreite und einer jeweiligen Ultraschallfrequenz eingestrahlt werden, wobei ein Pulsbreitenverhältnis der Ultraschallpulse in Abhängigkeit von der vorgegebenen Gewebetiefe und den Ultraschallfrequenzen eingestellt wird.

Bevorzugerweise werden dann die folgenden Verfahrensschritte von einer Steuereinrichtung durchgeführt:

a) Vorgeben einer Gewebetiefe für eine thermische Wirkung des Ultraschalls in dem Gewebe;

b) Vorgeben mindestens einer ersten und einer zweiten Ultraschallfrequenz, wobei jeder Ultraschallfrequenz eine jeweilige Eindringtiefe in dem Gewebe zugeordnet ist,

c) Festlegen des Pulsbreitenverhältnis in Abhängigkeit von der thermischen Wirkung und von den zugeordneten Eindringtiefen; und

d) Aktivieren und Deaktivieren des Einstrahlungsmittels zum sequentiellen Einstrahlen von Ultraschallpulsen mit dem festgelegten Pulsbreitenverhältnis.

Es ist dabei bevorzugt jeder Ultraschallfrequenz eine jeweilige Eindringtiefe in dem Gewebe zugeordnet, und es werden zwei unterschiedliche Ultraschallfrequenzen derart gewählt, dass ein vorgegebener Behandlungstiefenbereich in dem Gewebe zwischen den zwei zugeordneten Eindringtiefen liegt.

Besonders bevorzugt wird ein Frequenzverhältnis der unterschiedlichen Ultraschallfrequenzen derart gewählt, dass eine vorgegebene Behandlungstiefe in dem Gewebe erreicht wird. Die erfindungsgemäße sequentielle Einstrahlung mehrerer Ultraschallpulse mit verschiedenen Frequenzen hat insbesondere den Vorteil, dass die erforderliche Ultraschallleistung, um während einer Therapie mehrere Behandlungstiefen zu erreichen, praktisch nicht erhöht werden muss. Dies ist der Fall, wenn lediglich eine einzige fest vorgegebene Ultraschallfrequenz gepulst eingestrahlt wird.

Vorzugsweise werden der thermische Wirkungsparameter und der mechanische Wirkungsparameter, das Tastverhältnis, das Frequenzverhältnis und/oder die Behandlungstiefe über eine Anzeige dargestellt.

In einer besonders bevorzugten Ausführungsform der Vorrichtung werden jeweilige Tastverhältnisse, Pulsbreiten, Frequenzverhältnisse, Amplituden und/oder Gewebearten in einer Datenbank abgespeichert. Damit erlaubt die erfindungsgemäße Vorrichtung eine besonders einfache und gezielte Behandlung gemäß den therapeutischen Vorgaben, die im Wesentlichen die gewünschte thermische und mechanische Wirkung umfassen und gegebenenfalls von dem jeweiligen zu behandelnden Körperteil abhängen.

Dabei ist bevorzugt mindestens ein Anzeigemittel für die eingestellte mechanische Wirkung und/oder die thermische Wirkung vorgesehen. Ein beispielsweise als Bargraph ausgeführtes Anzeigemittel kann dem Therapeuten so zuverlässig die eingestellten bzw. vorgegebenen mechanischen und thermischen Wirkungsparameter anzeigen, die zu dem intern bestimmten Tastund/oder Frequenzverhältnis der Ultraschallpulse führen. Es wird somit eine besonders einfache und anschauliche Bedienung eines entsprechenden Ultraschallgerätes ermöglicht.

Vorzugsweise ist der Ultraschallkopf als Mehrfrequenzkopf ausgeführt.

In einer bevorzugten Ausführungsform ist eine an die Steuereinrichtung gekoppelte Speichereinrichtung vorgesehen, in der für eingestellte mechanische Wirkungen Therapieformen und Gewebearten, Tastverhältnisse und Ultraschallfrequenzangaben abgespeichert vorliegen. Es ist daher möglich, für jede Kombination von gewünschter thermischer und mechanischer Wirkung einen entsprechenden Parametersatz hinsichtlich der Tastverhältnisse, Frequenzen und möglicherweise weiterer Parameter zu programmieren. Dabei können einerseits auch aus Versuchsreihen gewonnene Erfahrungswerte verwendet werden, oder alternativ berechnet die Steuereinrichtung gemäß eines vorgegebenen Bestimmungsalgorithmus das Tastverhältnis. Eine bevorzugte Zuordnung der thermischen und mechanischen Wirkung auf ein Tastverhältnis sieht vor, dass das Tastverhältnis proportional zum Verhältnis zwischen dem mechanischen und dem thermischen Wirkungsparameter ist.

Die Erfindung betrifft ferner ein Computerprogrammprodukt mit einem auf einem Speichermittel maschinenlesbar gespeicherten Computerprogramm, welches auf einem Computer die Durchführung der entsprechenden Verfahren veranlasst, und wobei der Computer über eine Schnittstelle entsprechende Steuersignale zur Steuerung des Einstrahlungsmittels ausgibt. Ein Computerprogrammprodukt kann beispielsweise eine Diskette, CD-ROM oder ein sonstiges Speichermedium sein, welches in codierter Form die erfindungsgemäßen Verfahrensschritte zur computerimplementierten Ausführung bereitstellt.

Weitere vorteilhafte Ausgestaltungen und Weiterbildungen der Erfindung sind Gegenstand der Unteransprüche sowie der im Folgenden unter Bezugnahme auf die Figuren beschriebenen Ausführungsbeispiele. Es zeigt dabei:

Figur 1:     ein Blockdiagramm einer erfindungsgemäßen Vorrichtung zur Einstrahlung von Ultraschall;

Figur 2:     beispielhafte Signalformen von Ultraschallpulsen;

Figur 3:     ein Ablaufdiagramm des erfindungsgemäßen Verfahrens zum Einstrahlen von Ultraschall; und

Figur 4:     eine schematische Darstellung von erfindungsgemäß erzeugten Ultraschallpulsen.

In den Figuren sind, sofern nichts Anderes angegeben ist, gleiche bzw. funktionsgleiche Elemente mit denselben Bezugszeichen versehen worden.

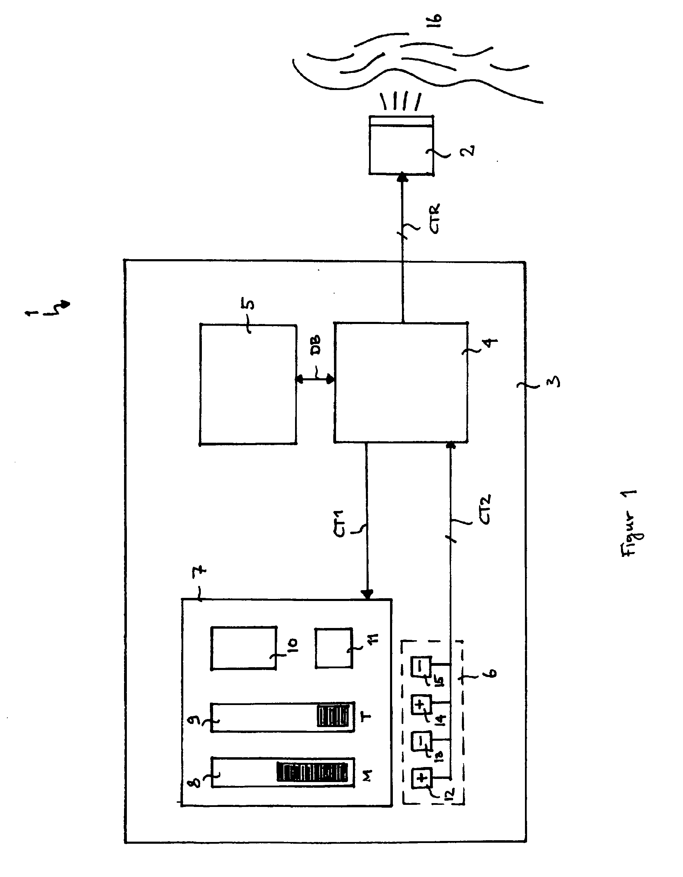

Die Figur 1 zeigt ein Blockschaltbild für eine erfindungsgemäße Vorrichtung zur Einstrahlung von Ultraschall. Die Vorrichtung 1 weist in dem hier dargestellten Ausführungsbeispiel einen Ultraschallkopf 2 auf, der an ein Steuergerät 3 angeschlossen ist. Das Steuergerät 3, welches beispielsweise computerimplementiert ausgeführt sein kann, liefert Steuersignale CTR an den Ultraschallkopf 2, welcher entsprechend Ultraschall in ein Gewebe 16 abstrahlt.

Das Steuergerät weist eine Steuereinrichtung 4 auf, welche an einen Speicher 5 über einen geeigneten Bus DB gekoppelt ist. Es ist ferner eine Eingabeeinrichtung 6 vorgesehen, mittels derer, beispielsweise durch einen Therapeuten, ein gewünschter mechanischer Wirkungsparameter und ein gewünschter thermischer Wirkungsparameter für die jeweilige Ultraschalltherapie eingebbar ist. Eine Anzeigeeinrichtung 7 stellt beispielsweise über Bargraphen 8, 9 die eingestellten Wirkungsparameter dar und verfügt über weitere Anzeigen oder Displays 10, 11, mittels derer beispielsweise eine Eindringtiefe, Kopplung der Ultraschallwellen an das Gewebe 16 oder weitere Einzelheiten zu der konkreten Ultraschalltherapie angezeigt werden. Die Anzeigeeinrichtung 7 und Eingabeeinrichtung 6 sind über geeignete Steuerleitungen CT1, CT2 an die Steuereinrichtung 4 gekoppelt. Die beiden Bargraphen 8, 9 zeigen hier dem Bediener bzw. Therapeuten die eingestellte thermische biologische Wirkung T auf das zu therapierende Gewebe 16 in Form der effektiven Leistung $P_{eff}$ in geeigneten Einheiten, wie beispielsweise W/cm$^2$ an, wie auch die eingestellte mechanische biologische Wirkung M auf das zu bestrahlende Gewebe 16 in Form der Amplitude der Leistung $P_{peak}$, ebenfalls in W/cm$^2$. Die benötigte Eindringtiefe kann ebenso eingestellt werden, wie auch die Behandlungsdauer insgesamt.

Abhängig von den Wirkungsparametern $P_{peak}$ und $P_{eff}$ berechnet oder bestimmt die Steuereinrichtung 4 günstige Tastverhältnisse für die gepulste Abgabe von Ultraschallwellen einer ebenfalls vorgegebenen Frequenz. Dabei sind beispielsweise in dem Speicher 5 Zuordnungstabellen abgelegt, die Kombinationen von Ultraschallfrequenzen, der effektiven Leistung $P_{eff}$ und der maximalen Amplitude der Leistung $P_{peak}$ ein jeweiliges Tastverhältnis T1/T2 zuordnen.

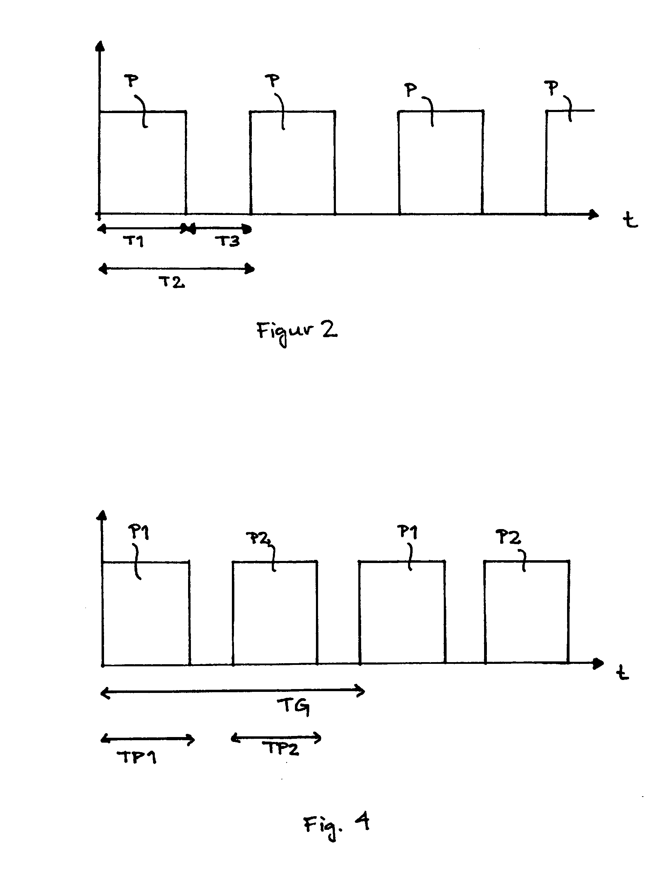

In der Figur 2 ist beispielhaft eine mögliche zeitliche Abfolge von Ultraschallpulsen P dargestellt. Es werden Ultraschallpulse P mit einer Periodenlänge T2 durch den Ultraschallkopf 2 abgestrahlt, wobei während einer Zeit T1 ein Ultraschallpuls der vorgegebenen Frequenz, beispielsweise 800 MHz abgibt. Darauf folgt ein Zeitraum T3 ohne Ultraschallabstrahlung. Das Verhältnis T1/T2 gibt das Tastverhältnis an.

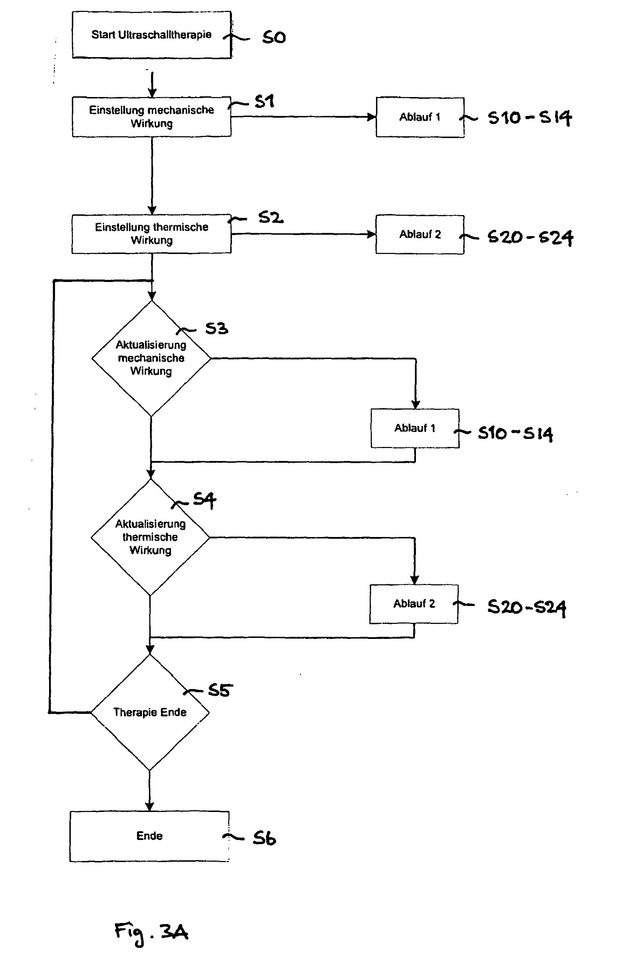

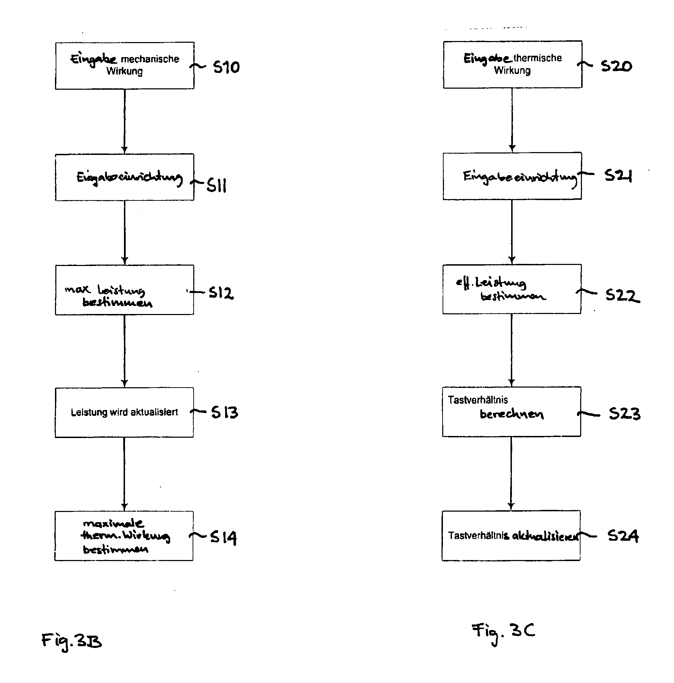

In den Figuren 3A-3C ist ein Ablaufdiagramm des erfindungsgemäßen Verfahrens dargestellt. Die Verfahrens- und Berechnungsschritte werden im Wesentlichen von der Steuereinrichtung 4 des Steuergerätes 3 durchgeführt, welches in der Figur 3 dargestellt ist. Es sind in der Abfolge S0-S6 die wesentlichen Schritte zur Durchführung einer therapeutischen Ultraschallanwendung dargestellt.

Im Schritt S0 wird die Ultraschalltherapie gestartet. Der Schritt S1 umfasst die Einstellung der gewünschten mechanischen Wirkung, welche durch die Ultraschalltherapie erzielt werden soll. Die Figur 3B zeigt die entsprechenden Schritte S10-S14. Der Therapeut gibt im Schritt S10 die mechanische Wirkung M über die Taster 12, 13 der Eingabeeinrichtung 6 ein (Schritt S11), was gleichzeitig über die Anzeige 7 als Bargraph 8 qualitativ dargestellt wird. Der Therapeut gibt demnach die Amplitude $P_{peak}$ der einzustrahlenden Leistung ein. Daraus wird im Schritt S12 die sich daraus ergebende Ultraschallleistung bestimmt und im Schritt S13 aktualisiert. Im Schritt S14 wird die maximal mögliche thermische Wirkung bestimmt, welche sich aus der Effektivleistung $P_{eff}$ ergibt, die wiederum von der eingestellten Amplitude $P_{peak}$ abhängt.

Im Folgeschritt S2 geschieht die Einstellung der gewünschten thermischen Wirkung T anhand der durch den Therapeuten ausgewählten abzustrahlenden Leistung in das Gewebe. Im Schritt S20 erfolgt die Eingabe der gewünschten thermischen Wirkung T als thermischer Wirkungsparameter in Form von $P_{eff}$. Dies wird von der Eingabeeinrichtung 6 im Schritt S21 der Steuereinrichtung 4 übermittelt. Aus der eingestellten gewünschten Leistung bzw. Dosis wird die entsprechende effektive Leistung bestimmt (Schritt S22).

Im Folgeschritt S23 ermittelt die Steuereinrichtung 4 ein entsprechendes Tastverhältnis T1/T2 für gepulste Ultraschalleinstrahlung. Dabei entspricht das Verhältnis $P_{peak}/P_{eff}$ dem Tastverhältnis T1/T2. Die entsprechende Zuordnung von thermischer und mechanischer Wirkung T, M zu dem Tastverhältnis T1/T2 unter Berücksichtigung der jeweiligen Ultraschallfrequenz ist in dem Speicher 5 abgelegt. Schließlich wird im Schritt S24 die derartig ermittelte Kombination von Ultraschallfrequenz und Tastverhältnis T1/T2 aktualisiert bzw. über Steuersignale CTR dem Ultraschallkopf 2 übermittelt.

Es erfolgt nun also eine gepulste Einstrahlung von Ultraschall, die exakt den therapeutischen Vorstellungen des Bedieners entspricht, ohne dass der Bediener oder Therapeut besondere Überlegungen anstellen muss, wie die Pulsabfolge hinsichtlich des Tastverhältnisses T1/T2 eingestellt werden muss.

Wird während der Therapie die mechanische Wirkung M in einem Schritt S3 durch Veränderung des eingestellten Wirkungsparameters verändert, wird in der Folge auch die gewünschte thermische Wirkung T durch Veränderung des Wirkungsparameters verändert bzw. aktualisiert, und es erfolgt erneut ein Ablauf, wie der in der Figur 3C dargestellt ist.

Wurde das zu therapierende Gewebe entsprechend bestrahlt, wird die Therapie beendet (Schritt S5) und die Ultraschalleinstrahlung eingestellt (Schritt S6).

Die in der Figur 3A dargestellten Schritte, insbesondere die Aktualisierungen der jeweiligen mechanischen und thermischen Wirkung (Schritte S3, S4) können auch in programmierter Form vorliegen, sodass die Steuereinrichtung 4 einen entsprechenden Therapieablauf aus dem Speicher 5 ausliest und Ultraschalleinstrahlungen über den Ultraschallkopf 2 vornimmt. Insofern kann im Schritt S5 eine Überprüfung erfolgen, ob alle Therapieschritte bereits durchgeführt sind oder die Schritte S3-S4 erneut durchlaufen werden sollen.

Neben der automatischen Bestimmung und Einstellung des Tastverhältnisses T1/T2 aus den für Therapeuten aussagekräftigen Wirkungsparametern hinsichtlich der mechanischen und thermischen Wirkung M, T im Gewebe, sieht die Erfindung vor, durch Abgabe von Ultraschallpulsen mit verschiedenen Frequenzen eine besonders gute Tiefenwirkung zu erzielen bzw. einen vorgegebenen Bereich von Behandlungstiefen gezielt zu bestrahlen.

Die Eindringtiefe Z des Ultraschalls hängt im Wesentlichen von der ausgewählten Ultraschallfrequenz f ab und sinkt in der Regel mit steigender Frequenz. Man spricht von der sogenannte Halbwertstiefe, bei der die Intensität I(z) von Ultraschallstrahlung im Gewebe um die Hälfte abgefallen ist. Bei 800 KHz sinkt in einem Muskelgewebe die Ultraschallintensität nach etwa 2,9 cm auf die Hälfte. Bei 3 MHz beträgt diese Halbwertstiefe jedoch nur 0,77 cm. Der Intensitätsabfall in Abhängigkeit von der Gewebetiefe weist meist einen exponentiellen Zusammenhang auf:

$$I(z) = I_0 \, e^{-\alpha \cdot f \cdot z}, \qquad\qquad (Gl. 1)$$

wobei $I_0$ ein Effektivwert bei der Tiefe z = 0 ist, $\alpha$ ein Abklingparameter, der gewebeabhängig ist, und f die Ultraschallfrequenz bezeichnet.

Für die Wärmeerzeugung in einer Gewebetiefe z durch eingestrahlte Ultraschallwellen lässt sich zeigen, dass für jede gewünschte zu erwärmende Gewebetiefe eine optimale Ultraschallfrequenz existiert. Die erzeuge Wärme hängt von dem Leistungsdichteabfall in Abhängigkeit von der Gewebetiefe und der Ultraschallfrequenz ab. Dabei ist es möglich, dass bis zu einer gewissen Tiefe, beispielsweise 2 cm bei Skelettmuskulatur, hohe Frequenzen mehr Leistungen in Wärme umwandeln als Niedrigere. Ab 2 cm Gewebetiefe erzeugen allerdings tiefere Frequenzen mehr Wärme als Höhere. Es lässt sich also durch Einstellen der Ultraschallfrequenz die Gewebetiefe mit der größten Wärmeerzeugung einstellen.

In der Regel haben tiefere Frequenzen zwischen 0,5 und 1,5 MHz in größerer Tiefe ihre optimale Wirkung hinsichtlich der Wärmeerzeugung. Bei Frequenzen ab 3 MHz liegt die optimale Tiefe in der Größenordnung von 1 cm und ist nur noch gering von der Frequenz abhängig. Untersuchungen der Anmelderin haben ergeben, dass ein günstiger Frequenzbereich zwischen 0,7 und 2,5 MHz zur Einstellung der durch Ultraschall erzeugten Wärme günstig ist.

Üblicherweise sind Ultraschallschwinger bzw. Ultraschallköpfe zum Abstrahlen von Ultraschallwellen auf eine einzige Ultraschallfrequenz ausgelegt. Es lassen sich meist allenfalls ganzzahlige Vielfache dieser Grundfrequenz des Ultraschallkopfes abstrahlen. Typische Frequenzen sind Vielfache von 800 KHz, also 1,6 und 2,4 MHz. Die optimalen Tiefen für Wärmeerzeugung sind bei 0,8 MHz 4,17 cm und bei 2,4 MHz 1,39 cm. Um eine zwischen diesen Tiefen liegende Gewebeschicht effizient mit Wärme durch Ultraschallanwendung zu behandeln, müsste jedoch ein Zwischenwert zwischen 0,8 und 2,4 MHz realisierbar sein. Dies ist in der Regel nicht möglich.

Die Erfindung sieht nun vor, um die optimale Tiefe zur Wärmeentwicklung durch Ultraschall nachzubilden, abwechselnd Ultraschallpulse mit der Grundfrequenz 0,8 und der dreifachen Frequenz, also 2,4 MHz einzustrahlen. Dadurch wird in einer Tiefe zwischen den Grenztiefen 1,39 cm und 4,17 cm eine optimale Tiefe zur Umsetzung in Wärme realisiert, sofern die Umschaltung zwischen den eingestrahlten Frequenzen schneller erfolgt, als die Wärmezeitkonstante in dem Gewebe. Die Wärmezeitkonstante gibt die Zeit vor, in der die Temperatur eines Wärmespeichers, bzw. hier eines Gewebebereiches, durch Wärmeverluste nur noch etwa 63 % der Ausgangstemperatur beträgt.

Um beispielsweise eine optimale Tiefe zur Wärmeerzeugung von 2,78 cm nachzubilden, wird erfindungsgemäß abwechselnd zum Beispiel eine Sekunde lang ein Ultraschallpuls von 0,8 MHz eingestrahlt, gefolgt von einem eine Sekunde langen Puls mit 2,4 MHz. Daraus ergibt sich die optimale Tiefe von 2,78 cm = (4,17 cm + 1,39 cm)/2. In diesem einfachen Fall ist für jeden Ultraschallpuls das Tastverhältnis T1/T2 = 1. Es lässt sich eine gewünschte Behandlungstiefe prinzipiell gemäß der folgenden Gleichung einstellen:

$$Z(TG) = \frac{1}{TG} \ [Z(TP1) \cdot TP1 + Z(TP2) \cdot TP2]. \qquad (Gl. \ 2)$$

Dabei lautet Z(TG) die gewünschte Behandlungstiefe, TG = TP1 + TP2 ist die Dauer eines erfindungsgemäßen Ultraschallzyklus, TP1 und TP2 die Pulslängen der beiden Ultraschallpulse P1, P2 mit jeweils einer Frequenz f1, f2. Z(TP1) ist die optimale Wirktiefe, also die Gewebetiefe, in der eine maximale Leistungsdichte in Wärme umgesetzt wird, für den Ultraschallpuls P1, und analog Z(TP2) für TP2.

In der Figur 4 sind entsprechende erfindungsgemäß erzeugte Ultraschallpulsfolgen dargestellt. Es sind abwechselnd Ultraschallpulse P1 und P2 mit einer jeweiligen Pulsdauer von TP1 und einer Frequenz f1 und f2 vorgesehen, um eine Behandlungstiefe gemäß der Gleichung 2 zu erzielen. In der Figur 4 ist ferner ein jeweiliges Tastverhältnis für die Ultraschallpulse P1, P2 angenommen, das ungleich 1 ist.

Somit lässt sich erfindungsgemäß sowohl die Behandlungstiefe präzise wie auch die gewünschte thermische und mechanische Wirkung für die Therapie einstellen. Um bei einer durch den Benutzer vorgegebenen Behandlungstiefe und meist durch den Ultraschallkopf vorgegebene Frequenzen eine Therapie durchzuführen, muss der Bediener an dem Steuergerät lediglich die Wirkungsparameter und Behandlungstiefe eingeben. Entsprechende Ultraschallpulslängen, Frequenzen und Tastverhältnisse ermittelt das erfindungsgemäße Steuergerät 3 dann selbstständig.

Die vorliegende Erfindung ermöglicht es insbesondere, auf besonders einfache Weise, die durch den Therapeuten gestellten Anforderungen an eine Ultraschalltherapie umzusetzen. Das automatische Bestimmen des Tastverhältnisses der einzustrahlenden Ultraschallpulse sowie auch der Pulsdauer und Frequenz, um die gewünschte Behandlungstiefe festzulegen, erfolgt selbsttätig. Das Verfahren des sequentiellen Einstrahlens von Ultraschallpulsen verschiedener Frequenz ermöglicht es, stufenlos Behandlungstiefen festzulegen, auch wenn nur eine begrenzte Anzahl von verschiedenen Ultraschallfrequenzen bereitstehen. Somit kann auch die Anzahl von benötigten Ultraschallköpfen in einer Therapiepraxis reduziert werden.

Obwohl die vorliegende Erfindung anhand von bevorzugten Ausführungsbeispielen näher erläutert wurde, ist sie nicht darauf beschränkt, sondern auf vielfältige Art und Weise modifizierbar. Die dargestellten Signalformen sind lediglich beispielhaft zu verstehen. Es sind selbstverständlich abweichende Ultraschallfrequenzen einsetzbar und verschiedene bekannte Anzeigemittel für die Wirkungsparameter zu verwendbar. Als Wirkungsparameter kommen auch von $P_{peak}$ und $P_{eff}$ abgeleitete Größen in Frage, wie zum Beispiel die jeweilige abgestrahlte Energie, die in J/cm$^2$ angegeben wird. Insbesondere kann das erfindungsgemäße Steuergerät auch direkt Ultraschallsignale generieren, die erfindungsgemäße Signalformen aufweisen. Insofern können die Steuersignale auch selbst als Ultraschallsignale verstanden werden. Selbstverständlich ist auch eine computerimplementierte Ausführung der Erfindung als Computerprogramm möglich.

Bezugszeichenliste

| S1-S24 | Verfahrensschritte |
| CT1, CT2, CTR | Steuersignale |
| DB | Datenbus |
| M | mechanischer Wirkungsparameter |
| T | thermischer Wirkungsparameter |
| P1, P2 | Ultraschallpuls |
| T1, T2, T3 | Zeitdauer |
| TP1, TP2 | Pulsdauer |
| TG | Zyklusdauer |

| 1 | Vorrichtung zur Erzeugung von Ultraschall |
| 2 | Ultraschallkopf |
| 3 | Steuergerät |
| 4 | Steuereinrichtung |
| 5 | Speicher |
| 6 | Eingabeeinrichtung |
| 7 | Anzeigeeinrichtung |
| 8, 9 | Bargraphen |
| 10, 11 | Anzeigen |
| 12, 13 | Taster |
| 14, 15 | Taster |
| 16 | Gewebe |

**Patentansprüche**

1. Vorrichtung (1) zur Einstrahlung von Ultraschall in ein Gewebe (16), mit:

   - einer Eingabeeinrichtung (6) zum Einstellen zumindest einer mechanischen Wirkung (M), einer thermischen Wirkung (T) und/oder einer Behandlungstiefe (z) des Ultraschalls in dem Gewebe (16);
   - mindestens einem Ultraschallkopf (2), welcher in Abhängigkeit von Steuersignalen (CTR) aktiviert und deaktiviert wird und Ultraschallpulse abstrahlt;

   **gekennzeichnet durch**
   eine Steuereinrichtung (4), welche derart ausgestaltet ist, dass bei einer vorgegebenen thermischen Wirkung (T) und einer vorgegebenen mechanischen Wirkung (M) in dem Gewebe (16) die Steuereinrichtung (4) derart entsprechende Steuersignale (CTR) erzeugt, dass von dem Ultraschallkopf (2) nacheinander Ultraschallpulse (P) mit einer jeweiligen Pulsbreite (T1) eingestrahlt werden, und ein Tastverhältnis (T1/T2) der Ultraschallpulse (P) in Abhängigkeit von der thermischen und der mechanischen Wirkung (T, M) des Ultraschalls eingestellt wird.

2. Vorrichtung (1) nach Anspruch 1, **dadurch gekennzeichnet, dass** die Steuereinrichtung (4) folgende Verfahrensschritte durchgeführt:

   a) Vorgeben eines mechanischen Wirkungsparameters ($Pp_{eak}$) für die mechanische Wirkung;
   b) Vorgeben eines thermischen Wirkungsparameter ($P_{eff}$) für die thermische Wirkung;
   c) Festlegen des Tastverhältnisses (T1/T2) in Abhängigkeit von dem thermischen Wirkungsparameter ($P_{eff}$) und von dem mechanischen Wirkungsparameter ($P_{peak}$); und
   d) Erzeugen von Steuersignalen (CTR) zum Aktivieren und Deaktivieren des Einstrahlungsmittels zum Einstrahlen von Ultraschallpulsen mit dem festgelegten Tastverhältnis (T1/T2).

3. Vorrichtung (1) nach Anspruch 1 oder 2, **dadurch gekennzeichnet, dass** eine Amplitude ($P_{peak}$) der abgegebenen Ultraschallleistung als mechanischer Wirkungsparameter verwendet wird.

4. Vorrichtung (1) nach mindestens einem der Ansprüche 1 bis 3, **dadurch gekennzeichnet, dass** eine effektiv abgegebene Ultraschallleistung ($P_{eff}$) als thermischer Wirkungsparameter verwendet wird.

5. Vorrichtung (1) nach mindestens einem der Ansprüche 1 bis 4, **dadurch gekennzeichnet, dass** die Steuereinrichtung (4) aus der vorgegebenen mechanischen Wirkung eine maximale thermische Wirkung ermittelt.

**6.** Vorrichtung (1) nach mindestens einem der Ansprüche 1 bis 5, **dadurch gekennzeichnet, dass** die Ultraschallpulse unterschiedliche Ultraschallfrequenzen (f1, f2) aufweisen.

**7.** Vorrichtung (1) nach mindestens einem der Ansprüche 1 bis 6, **dadurch gekennzeichnet, dass** das Tastverhältnis zu Eins gesetzt ist.

**8.** Vorrichtung (1) nach mindestens einem der Ansprüche 1 bis 7, **dadurch gekennzeichnet, dass** die Steuereinrichtung (4) gemäß eines vorgegebenen Bestimmungsalgorithmus das Tastverhältnis (T1/T2) ermittelt.

**9.** Vorrichtung (1) zur Einstrahlung von Ultraschall in ein Gewebe (16), mit:

- einer Eingabeeinrichtung (6) zum Einstellen zumindest einer mechanischen Wirkung (M), einer thermischen Wirkung (T) und/oder einer Behandlungstiefe (z) des Ultraschalls in dem Gewebe (16);
- mindestens einem Ultraschallkopf (2), welcher in Abhängigkeit von Steuersignalen (CTR) aktiviert und deaktiviert wird und Ultraschallpulse abstrahlt;

**gekennzeichnet durch**
eine Steuereinrichtung (4), welche derart ausgestaltet ist, dass bei einer vorgegebenen thermischen Wirkung (T) und in einer vorgegebenen Gewebetiefe (z) in dem Gewebe (16) die Steuereinrichtung (4) derart entsprechende Steuersignale (CTR) erzeugt, dass von dem Ultraschallkopf (2) nacheinander Ultraschallpulse (P1, P2) mit einer jeweiligen Pulsbreite (TP1, TP2) und unterschiedlichen Ultraschallfrequenzen (f1, f2) eingestrahlt werden, wobei ein Pulsbreitenverhältnis (TP1/TP2) der Ultraschallpulse (P1, P2) in Abhängigkeit von der vorgegebenen Gewebetiefe (z) und den Ultraschallfrequenzen (f1, f2) eingestellt wird.

**10.** Vorrichtung (1) nach Anspruch 9, **dadurch gekennzeichnet, dass** die Steuereinrichtung (4) folgende Verfahrensschritte durchgeführt:

a) Vorgeben der Gewebetiefe (Z(TG)) für die thermische Wirkung (T) des Ultraschalls in dem Gewebe (16);
b) Vorgeben mindestens einer ersten und einer zweiten Ultraschallfrequenz (f1, f2), wobei jeder Ultraschallfrequenz (f1, f2) eine jeweilige Eindringtiefe (Z(TP1), Z(TP2)) in dem Gewebe (16) zugeordnet ist,
c) Festlegen des Pulsbreitenverhältnis (TP1/TP2) in Abhängigkeit von der thermischen Wirkung (T) und von den zugeordneten Eindringtiefen (Z(TP1), Z(TP2)); und
d) Aktivieren und Deaktivieren des Einstrahlungsmittels (2) zum sequentiellen Einstrahlen von Ultraschallpulsen (P1, P2) mit dem festgelegten Pulsbreitenverhältnis (TP1/TP2).

**11.** Vorrichtung (1) nach mindestens einem der Ansprüche 1 bis 10, **dadurch gekennzeichnet, dass** mindestens ein Anzeigemittel (8, 9, 10, 11) für die eingestellte mechanische Wirkung, die thermische Wirkung und/oder der Behandlungstiefe (z) vorgesehen ist.

**12.** Vorrichtung (1) nach mindestens einem der Ansprüche 1 bis 11, **dadurch gekennzeichnet, dass** der Ultraschallkopf (2) als Mehrfrequenzkopf ausgeführt ist.

**13.** Vorrichtung (1) nach mindestens einem der Ansprüche 1 bis 12, **dadurch gekennzeichnet, dass** eine an die Steuereinrichtung (4) gekoppelte Speichereinrichtung (5) vorgesehen ist, welche für eingestellte mechanische und thermische Wirkungen, Therapieformen und Gewebearten, Tastverhältnisse und Ultraschallfrequenzangaben vorhält.

**14.** Computerprogrammprodukt mit einem auf einem Speichermittel maschinenlesbar gespeicherten Computerprogramm, welches auf einem Computer die Durchführung eines Verfahrens zum Ansteuern eines Einstrahlungsmittels (2) für die Einstrahlung von Ultraschall in ein Gewebe (16) mit einer vorgegebenen thermischen Wirkung (T) und einer vorgegebenen mechanischen Wirkung (M) in dem Gewebe (16) veranlasst, bei dem nacheinander Ultraschallpulse (P) mit einer jeweiligen Pulsbreite (T1) eingestrahlt werden, und wobei ein Tastverhältnis (T1/T2) der Ultraschallpulse (P) in Abhängigkeit von der thermischen und der mechanischen Wirkung (T, M) des Ultraschalls eingestellt wird, wobei der Computer über eine Schnittstelle Steuersignale für das Einstrahlungsmittel ausgibt.

**15.** Computerprogrammprodukt mit einem auf einem Speichermittel maschinenlesbar gespeicherten Computerprogramm, welches auf einem Computer die Durchführung eines Verfahrens zum Ansteuern eines Einstrahlungsmittels (2) für die Einstrahlung von Ultraschall in ein Gewebe (16) mit einer vorgegebenen thermischen Wirkung (T) in einer vorgegeben Gewebetiefe (z) in dem Gewebe (16) veranlasst, wobei nacheinander Ultraschallpulse (P1, P2) mit einer jeweiligen Pulsbreite (TP1, TP2) und unterschiedlichen Ultraschallfrequenzen (f1, f2) eingestrahlt werden, und wobei ein Pulsbreitenverhältnis (TP1/TP2) der Ultraschallpulse (P1, P2) in Abhängigkeit von der vorgegebenen Gewebetiefe (z) und den Ultraschallfrequenzen (f1, f2) eingestellt wird, wobei der Computer über eine Schnittstelle Steuersignale für das Einstrahlungsmittel ausgibt.

**Claims**

1. Apparatus (1) for irradiating ultrasound into tissue (16), comprising:

   - an input unit (6) for setting at least one mechanical effect (M), one thermal effect (T) and/or one ultrasound treatment depth (z) within the tissue (16);
   - at least one ultrasound transducer head (2) being activated and deactivated as a function of control signals (CTR) and emitting ultrasonic pulses;

   said apparatus being **characterized by**
   a control unit (4) configured in such a way that, at a predetermined thermal effect (T) and a predetermined mechanical effect (M) within said tissue (16), said control unit (4) generates corresponding control signals (CTR) so that ultrasonic pulses (P) having a respective pulse width (T1) are irradiated successively by said ultrasound transducer head (2), and that a pulse duty factor (T1/T2) of said ultrasonic pulses (P) is set as a function of said ultrasonic thermal and ultrasonic mechanical effect (T, M).

2. Apparatus (1) according to claim 1, **characterized in that** said control unit (4) performs the following steps:

   a) Setting a mechanical effective parameter ($P_{peak}$) for the mechanical effect;
   b) Setting a thermal effective parameter ($P_{eff}$) for the thermal effect;
   c) Determining said pulse duty factor (T1/T2) as a function of said thermal effective parameter ($P_{eff}$) and said mechanical effective parameter ($P_{peak}$); and
   d) Generating control signals (CTR) to activate and deactivate said irradiation means for irradiation of ultrasonic pulses having said predetermined pulse duty factor (T1/T2).

3. Apparatus (1) according to claim 1 or 2, **characterized in that** an amplitude ($P_{peak}$) of the outputted ultrasonic power is used as a mechanical effective parameter.

4. Apparatus (1) according to at least one of the claims 1 to 3, **characterized in that** an effectively outputted ultrasonic power ($P_{eff}$) is used as a thermal effective parameter.

5. Apparatus (1) according to at least one of the claims 1 to 4, **characterized in that** said control unit (4) determines a maximum thermal effect from the predetermined mechanical effect.

6. Apparatus (1) according to at least one of the claims 1 to 5, **characterized in that** said ultrasonic pulses have different ultrasonic frequencies (f1, f2).

7. Apparatus (1) according to at least one of the claims 1 to 6, **characterized in that** said pulse duty factor is set to be equal to one.

8. Apparatus (1) according to at least one of the claims 1 to 7, **characterized in that** said control unit (4) determines said pulse duty factor (T1/T2) according to a given determination algorithm.

9. Apparatus (1) for irradiating ultrasound into tissue (16), comprising:

   - an input unit (6) for setting at least one mechanical effect (M), one thermal effect (T) and/or one ultrasound treatment depth (z) within the tissue (16);
   - at least one ultrasound transducer head (2) being activated and deactivated as a function of control signals (CTR) and emitting ultrasonic pulses;

   said apparatus being **characterized by**

a control unit (4) configured in such a way that, at a predetermined thermal effect (T) and at a predetermined tissue depth (z) within said tissue (16), said control unit (4) generates corresponding control signals (CTR) so that ultrasonic pulses (P1, P2) having a respective pulse width (TP1, TP2) and different ultrasonic frequencies (f1, f2) are irradiated successively by the ultrasound transducer head (2), wherein a pulse duty factor (TP1/TP2) of said ultrasonic pulses (P1, P2) is set as a function of said predetermined tissue depth (z) and said ultrasonic frequencies (f1, f2).

**10.** Apparatus (1) according to claim 9, **characterized in that** said control unit (4) performs the following steps:

    a) Setting said tissue depth (Z(TG)) for said thermal effect (T) of the ultrasound in said tissue (16);
    b) Setting at least one first and one second ultrasonic frequency (f1, f2), wherein a respective penetration depth (Z(TP1), Z(TP2) within said tissue (16) is assigned to each ultrasonic frequency (f1, f2);
    c) Determining said pulse duty factor (TP1/TP2) as a function of said thermal effect (T) and said assigned penetration depths ((Z(TP1), Z(TP2)); and
    d) Activating and deactivating said irradiation means (2) for sequential irradiation of ultrasonic pulses (P1, P2) having said predetermined pulse duty factor (TP1/TP2).

**11.** Apparatus (1) according to at least one of the claims 1 to 10, **characterized in that** there is provided at least one indicating means (8, 9, 10, 11) for said predetermined mechanical effect, said thermal effect and/or said treatment depth (z).

**12.** Apparatus (1) according to at least one of the claims 1 to 11, **characterized in that** said ultrasound transducer head (2) is configured as a multifrequency transducer head.

**13.** Apparatus (1) according to at least one of the claims 1 to 12, **characterized in that** there is provided a memory device (5) coupled to the control unit (4), said memory device (5) having in store forms of therapy and types of tissue, pulse duty factors and ultrasonic frequency designations for predetermined mechanical and thermal effects.

**14.** Computer program product having a machine readable computer program stored in a storage medium, in a computer said computer program initiating the execution of a method of driving irradiation means (2) for irradiating ultrasound into tissue (16) with a predetermined thermal effect (T) and a predetermined mechanical effect (M) within said tissue (16), in said method ultrasonic pulses (P) having a respective pulse width (T1) are irradiated successively, and wherein a pulse duty factor (T1/T2) of the ultrasonic pulses (P) is set as a function of said ultrasonic thermal and ultrasonic mechanical effect (T, M), wherein said computer outputs control signals for the irradiation means via an interface.

**15.** Computer program product having a machine readable computer program stored in a storage medium, in a computer said computer program initiating the execution of a method of driving irradiation means (2) for irradiating ultrasound into tissue (16) with a predetermined thermal effect (T) at a predetermined tissue depth (z) within said tissue (16), in said method ultrasonic pulses (P1, P2) having a respective pulse width (TP1, TP2) and different ultrasonic frequencies (f1, f2) are irradiated successively, and wherein a pulse duty factor (TP1/TP2) of the ultrasonic pulses (P1, P2) is set as a function of said predetermined tissue depth (z) and said ultrasonic frequencies (f1, f2), wherein said computer outputs control signals for the irradiation means via an interface.

**Revendications**

**1.** Dispositif (1) pour irradier des ultrasons dans un tissu (16), comprenant:

    - une unité d'entrée (6) pour fixer au moins un effet mécanique (M), un effet thermique (T) et/ou une profondeur (z) de traitement ultrasonique dans ledit tissu (16);
    - au moins une sonde ultrasonore (2) étant activée et désactivée en fonction de signaux de commande (CTR) et émettant des impulsions ultrasonores,

ledit dispositif étant **caractérisé par**
une unité de commande (4) arrangée de sorte que lors d'un effet thermique (T) prédéterminé et lors d'un effet mécanique (M) prédéterminé dans ledit tissu (16) ladite unité de commande (4) génère des signaux de commande (CTR) correspondants de manière que des impulsions ultrasonores (P) avec une largeur d'impulsions (T1) respective sont irradiées successivement par ladite sonde ultrasonore (2), et qu'un taux d'impulsions (T1/T2) desdites impulsions ultrasonores (P) est réglé en fonction dudit effet thermique et mécanique (T, M) dudit ultrason.

2. Dispositif (1) selon la revendication 1, **caractérisé en ce que** ladite unité de commande (4) exécute les étapes suivantes:

   a) Fixer un paramètre d'effet mécanique ($P_{peak}$) pour l'effet mécanique;
   b) Fixer un paramètre d'effet thermique ($P_{eff}$) pour l'effet thermique;
   c) Déterminer ledit taux d'impulsions (T1/T2) en fonction dudit paramètre d'effet thermique ($P_{eff}$) et dudit paramètre d'effet mécanique ($P_{peak}$); et
   d) Générer des signaux de commande (CTR) pour activer et désactiver ledit dispositif d'irradiation pour irradier des impulsions ultrasonores avec ledit taux d'impulsions (T1/T2) prédéterminé.

3. Dispositif (1) selon l'une des revendications 1 à 2, **caractérisé en ce qu'**une amplitude ($P_{peax}$) de la puissance ultrasonore de sortie est utilisée comme paramètre d'effet mécanique.

4. Dispositif (1) selon l'une quelconque des revendications 1 à 3, **caractérisé en ce qu'**une puissance ultrasonore ($P_{eff}$) effective de sortie est utilisée comme paramètre d'effet thermique.

5. Dispositif (1) selon l'une quelconque des revendications 1 à 4, **caractérisé en ce que** ladite unité de commande (4) détermine un effet thermique maximal à partir dudit effet mécanique prédéterminé.

6. Dispositif (1) selon l'une quelconque des revendications 1 à 5, **caractérisé en ce que** lesdites impulsions ultrasonores comportent des fréquences ultrasonores (f1, f2) différentes.

7. Dispositif (1) selon l'une quelconque des revendications 1 à 6, **caractérisé en ce que** ledit taux d'impulsions est amené égal à un.

8. Dispositif (1) selon l'une quelconque des revendications 1 à 7, **caractérisé en ce que** ladite unité de commande (4) détermine ledit taux d'impulsions (T1/T2) suivant un algorithme de définition prédéterminé.

9. Dispositif (1) pour irradier des ultrasons dans un tissu (16), comprenant:

   - une unité d'entrée (6) pour fixer au moins un effet mécanique (M), un effet thermique (T) et/ou une profondeur (z) de traitement ultrasonique dans ledit tissu (16);
   - au moins une sonde ultrasonore (2) étant activée et désactivée en fonction de signaux de commande (CTR) et émettant des impulsions ultrasonores,

   ledit dispositif étant **caractérisé par**
   une unité de commande (4) arrangée de sorte que lors d'un effet thermique (T) prédéterminé et dans une profondeur (z) de tissu prédéterminée dans ledit tissu (16) ladite unité de commande (4) génère des signaux de commande (CTR) correspondants de manière que des impulsions ultrasonores (P1, P2) avec une largeur d'impulsions (TP1, TP2) respective et avec des fréquences ultrasonores (f1, f2) différentes sont irradiées successivement par ladite sonde ultrasonore (2), un taux d'impulsions (TP1/TP2) desdites impulsions ultrasonores (P1, P2) étant réglé en fonction de ladite profondeur (z) de tissu prédéterminée et desdites fréquences ultrasonores (f1, f2).

10. Dispositif (1) selon la revendication 9, **caractérisé en ce que** ladite unité de commande (4) exécute les étapes suivantes:

    a) Fixer ladite profondeur (Z(TG))) de tissu pour l'effet thermique (T) du ultrason dans ledit tissu (16);
    b) Fixer au moins une première et une secondaire fréquence ultrasonore (f1, f2), une profondeur de pénétration (Z(TP1), Z(TP2)) respective dans ledit tissu (16) étant attribuée à chacune des fréquences ultrasonores (f1, f2);
    c) Déterminer ledit taux d'impulsions (TP1/TP2) en fonction dudit effet thermique (T) et desdites profondeurs de pénétration (Z(TP1), Z(TP2)); et
    d) Activer et désactiver ledit moyen d'irradiation (2) pour irradier des impulsions ultrasonores (P1, P2) avec ledit taux d'impulsions (TP1/TP2) prédéterminé.

11. Dispositif (1) selon l'une quelconque des revendications 1 à 10, **caractérisé en ce qu'**au moins un dispositif indicateur (8, 9, 10, 11) est arrangé pour indiquer l'effet mécanique prédéterminé, l'effet thermique prédéterminé et/ou la profondeur (z) de traitement prédéterminée.

**12.** Dispositif (1) selon l'une quelconque des revendications 1 à 11, **caractérisé en ce que** ladite sonde ultrasonore (2) est construite comme sonde à plusieurs fréquences.

**13.** Dispositif (1) selon l'une quelconque des revendications 1 à 12, **caractérisé en ce qu'**un dispositif de mémoire (5) couplé à ladite unité de commande (4) est arrangé, ledit dispositif de mémoire (5) tenant à disposition des formes thérapeutiques et des types de tissu, des taux d'impulsions et des indications de fréquence ultrasonore pour des effets mécaniques prédéterminés et thermiques prédéterminés.

**14.** Produit de programme informatique avec un programme informatique lisible par ordinateur et mémorisé sur un support de stockage, ledit programme informatique permettant à un ordinateur d'exécuter un procédé de commande d'un moyen d'irradiation (2) des ultrasons dans un tissu (16) avec un effet thermique (T) prédéterminé et un effet mécanique (M) prédéterminé dans ledit tissu (16), des impulsions ultrasonores (P) avec une largeur d'impulsions (T1) respective étant irradiées successivement, et un taux d'impulsions (T1/T2) desdites impulsions ultrasonores (P) étant réglé en fonction dudit effet thermique et mécanique (T, M) dudit ultrason, et ledit ordinateur sortant en output par un interface des signaux de commande pour ledit moyen d'irradiation.

**15.** Produit de programme informatique avec un programme informatique lisible par ordinateur et mémorisé sur un support de stockage, ledit programme informatique permettant à un ordinateur d'exécuter un procédé de commande d'un moyen d'irradiation (2) des ultrasons dans un tissu (16) avec un effet thermique (T) prédéterminé dans une profondeur (z) de tissu prédéterminée dans ledit tissu (16), des impulsions ultrasonores (P1, P2) avec une largeur d'impulsions (TP1, TP2) respective et avec des fréquences ultrasonores (f1, f2) différentes étant irradiées successivement, et un taux d'impulsions (TP1/TP2) desdites impulsions ultrasonores (P1, P2) étant réglé en fonction de ladite profondeur (z) de tissu prédéterminée et desdites fréquences ultrasonores (f1, f2), et ledit ordinateur sortant en output par un interface des signaux de commande pour ledit moyen d'irradiation.

Figur 1

Fig. 3A

Fig. 3C

Fig. 3B

P    P    P    P

t

T1    T3

T2

Figur 2

P1    P2    P1    P2

t

TG

TP1    TP2

Fig. 4

**IN DER BESCHREIBUNG AUFGEFÜHRTE DOKUMENTE**

**In der Beschreibung aufgeführte Patentdokumente**

- DE 10306795 A1
- US 20040082857 A